(19) Europäisches Patentamt / European Patent Office / Office européen des brevets

(11) **EP 3 666 906 A1**

(12) **EUROPEAN PATENT APPLICATION**

(43) Date of publication:
**17.06.2020 Bulletin 2020/25**

(51) Int Cl.:
***C12Q 1/6886*** (2018.01)

(21) Application number: **18382912.6**

(22) Date of filing: **11.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Consejo Superior De Investigaciones Científicas**
**28006 Madrid (ES)**

(72) Inventors:
• **LORENZO MARTIN, Luis Francisco**
  **37007 Salamanca (ES)**

• **GARCIA BUSTELO, Xosé Ramón**
  **37007 Salamanca (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

Remarks:
Claims 16 and 17 are deemed to be abandoned due to non-payment of the claims fees (Rule 45(3) EPC).

(54) **METHODS AND KITS FOR THE PROGNOSIS OF SQUAMOUS CELL CARCINOMAS (SCC)**

(57) Here, we describe a prognostic gene signature for squamous cell carcinoma. Based on the expression levels of the Rho guanine nucleotide exchange factor Vav2 and/or of a Vav2-induced transcriptional signature comprising 41 key genes, a robust prediction of patient survival can be made. Furthermore, this prognostic gene signature can be used to uncover some of the molecular mechanisms underlying squamous cell carcinoma, thus leading to the identification of new therapeutic targets for the development of novel treatments. Considering this, we believe the present findings to be endowed with potential commercial application.

**EP 3 666 906 A1**

## Description

## FIELD OF THE INVENTION

The invention relates to the field of cancer prognosis and more particularly, to methods for predicting the outcome of a Squamous Cell Carcinoma patient based on the expression levels of several genes and to specific prognostic signatures and algorithms, as well as to methods for selecting those patients who would benefit from adjuvant treatment after tumor resection, and kits for implementing those methods.

## STATE OF THE ART

Squamous Cell Carcinomas (SCC) are among the most prevalent cancers worldwide, with hundreds of thousands of new cases being diagnosed every year (American Society of Clinical Oncology, 2017). They arise from the epithelial lining of different organs giving rise to a diverse group of tumors, including skin (cSCC), head and neck (HNSCC), lung (NSCLC) and thyroid (SCTC) carcinomas. Besides their high incidence, most SCC subtypes are characterized by a poor prognosis, with a 5-year survival rate usually below 60% (American Society of Clinical Oncology, 2017). These figures have not improved significantly in the past decade, exposing a clear need for new therapeutic approaches targeting these cancers.

Despite their differences, different SCC subtypes have been shown to share common molecular features (Hoadley et al., 2014). Proliferation, adhesion, survival, motility and invasion are some cellular functions recurrently altered in SCCs (Dotto and Rustgi, 2016). These functions are known to represent the portfolio of Rho GTPases, some of which have been found deregulated in these cancers (Porter et al., 2016). However, given that these proteins are rarely mutated, the molecular mechanisms underlying such deregulation remain largely unknown.

In the present invention, we have explored the relevance of Rho guanine nucleotide exchange factors (GEFs) as causal agents of SCC carcinogenesis. In silico, in vitro and in vivo approaches have revealed that the GEF VAV2 plays a relevant role in SCC tumorigenesis and harbors potential therapeutic value. We have distilled these findings into a small gene signature with robust prognostic value, which can be used to stratify SCC patients according to disease outcome and survival.

## BRIEF DESCRIPTION OF THE INVENTION

The classification of cancer patients into different risk groups is one of the key parameters determining prognosis and clinical treatment. In the last decade, molecular approaches that help perform such classification have led to the development of marketable products. Among them, prognostic gene signatures have already proven their clinical utility, as in the well-known case of 'MammaPrint', a 70-gene collection currently used in early-stage breast cancer.

Here, we describe a prognostic gene signature for squamous cell carcinoma. Based on the expression levels of the Rho guanine nucleotide exchange factor VAV2 and/or at least one of the genes of a VAV2-induced transcriptional signature comprising 41 key genes, a robust prediction of patient survival can be made. Furthermore, this prognostic gene signature can be used to uncover some of the molecular mechanisms underlying squamous cell carcinoma, thus leading to the identification of new therapeutic targets for the development of novel treatments. Considering this, we believe the present findings to be endowed with potential commercial application.

Therefore, the inventors have identified several signatures of expressed genes with prognostic value in patients with squamous cell carcinoma. These signatures are associated with the gene expression levels, preferably the individual mRNA levels, of the Rho guanine nucleotide exchange factor *VAV2* and/or at least one, or preferably all, of the VAV2-induced transcriptional signature comprising the following 41 key genes [*ABCA9, ACPP, ANXA9, AREG, ARHGAP20, ATP1A2, AURKA, BCAT1, BIRC5, CAMK1D, CCL4, CDCA5, CDCA8, CILP, CKS1B, CXCL2, EXPH5, FSCN1, FYCO1, HAS3, HSPB6, IL24, LAMC2, MMP9, MYC, NEK2, NOLC1, ODC1, PKIB, PYGM, SCEL, SERPINE1, SLC20A1, SLC2A3, SLC6A4, SLC7A11, SLC7A5, SOCS3, SRM, SSBP2, TCEA3]* (from hereinafter "the VAV2-induced transcriptional signature")

The identified signatures preferably consist of an algorithm which combines the coefficient-weighted expression of the selected marker genes analyzed by any known method useful for the transcriptomic profiling such as expression microarrays. The levels of the expression of each gene are preferably measured by a gene-Score and the algorithm assigns a coefficient to each gene G-Score to get a final combined score that in the context of this invention is called Prognostic Index (PI) value.

These genes scores and the prognostic indicator have been shown to provide independent prognostic information for recurrence free survival (DFS) and overall survival (OS) in patients with squamous cell carcinoma. Indeed, as shown in Examples 1 to 3, the identified signatures are able to discriminate with high statistical significance a group of squamous cell carcinoma patients who have worse survival prospects from those having better survival prospects. This group of patients should be monitored more closely and managed more intensely, and may be potential candidates for adjuvant chemotherapy.

Accordingly, a first aspect of the present invention relates to a multi-platform method for determining the prognosis of squamous cell carcinoma patients, preferably selected from skin (cSCC) and head and neck (HNSCC) carcinomas, wherein said method comprises:

a) determining in a biological sample isolated from said subject the gene expression levels of the Rho guanine nucleotide exchange factor *VAV2* and/or, at least one, or preferably all, of the VAV2-induced transcriptional signature markers selected from the group consisting of:

- *ABCA9, ACPP, ANXA9, AREG, ARHGAP20, ATP1A2, AURKA, BCAT1, BIRC5, CAMK1D, CCL4, CDCA5, CDCA8, CILP, CKS1B, CXCL2, EXPH5, FSCN1, FYCO1, HAS3, HSPB6, IL24, LAMC2, MMP9, MYC, NEK2, NOLC1, ODC1, PKIB, PYGM, SCEL, SERPINE1, SLC20A1, SLC2A3, SLC6A4, SLC7A11, SLC7A5, SOCS3, SRM, SSBP2, TCEA3;*

b) calculating a combined score from the gene expression levels of the markers determined in the biological sample as defined in step a); and

c) classifying the subject as having good prognosis or poor prognosis based on the combined score.

In another aspect, the invention relates to a method for determining target gene expression levels by any known method useful for the transcriptomic profiling such as expression microarrays, wherein said method is suitable for carrying out the determination of the gene expression levels of the markers in the biological sample as defined in step a) of the prognosis method of the invention.

Additionally, the invention provides an *in vitro* method for selecting those subjects having squamous cell carcinoma, preferably selected from skin (cSCC) and head and neck (HNSCC) carcinomas, who are expected to benefit from an adjuvant treatment, wherein said method comprises:

a) classifying said subject according to the prognosis method as defined herein;
b) selecting for administration of an adjuvant treatment a subject classified in step a) as having poor prognosis.

The invention also relates to an *in vitro* method for predicting the efficacy of an adjuvant chemotherapy in a subject having squamous cell carcinoma, wherein said method comprises:

a) classifying said subject according to the prognosis method as defined herein; and
b) predicting the efficacy of said adjuvant chemotherapy according to the prognosis classification;

wherein classification of the subject in step a) as having poor prognosis is indicative of increased efficacy of the adjuvant chemotherapy.

Moreover, the invention provides an *in vitro* method for selecting an adjuvant treatment for a subject having squamous cell carcinoma, preferably selected from skin (cSCC) and head and neck (HNSCC) carcinomas, wherein said method comprises:

a) classifying said subject according to the prognosis method as defined herein; and
b) selecting an adjuvant treatment according to the prognosis classification;

wherein when the subject is classified in step a) as having poor prognosis then adjuvant chemotherapy is selected as adjuvant treatment.

In a further aspect, the invention relates to a data-processing apparatus comprising means for carrying out one or more of the steps of the methods as described herein.

In another aspect, the invention pertains to a computer program comprising instructions which, when the program is executed by a computer, cause the computer to carry out one or more of the steps of the methods of the invention. The invention also refers to a computer-readable storage medium having stored thereon such a computer program.

In an additional aspect, the present invention provides a method for treating a subject having squamous cell carcinoma, preferably selected from skin (cSCC) and head and neck (HNSCC) carcinomas, comprising administering to said patient a therapeutically effective amount of a treatment, typically an adjuvant treatment, wherein said treatment is selected according to the classification of said patient according to the prognosis method of the invention. Preferably, when said patient is classified as having poor prognosis the administered treatment comprises a platinum anticancer agent.

In another further aspect, the invention relates to a kit suitable for use in a method for the prognosis of a subject having head and neck squamous cell carcinoma as defined herein, wherein said kit comprises:

- an affinity reagent for a product of the gene expression, preferably for the mRNA, of the Rho guanine nucleotide exchange factor *VAV2* and/or, all of the markers of the VAV2-induced transcriptional signature; and
- optionally, further comprising cancer cells to be used as low and/or high expression controls;
- optionally, further comprising instructions for the use of said reagents in determining said gene expression levels in a biological sample isolated from a subject.

**BRIEF DESCRIPTION OF THE DRAWINGS**

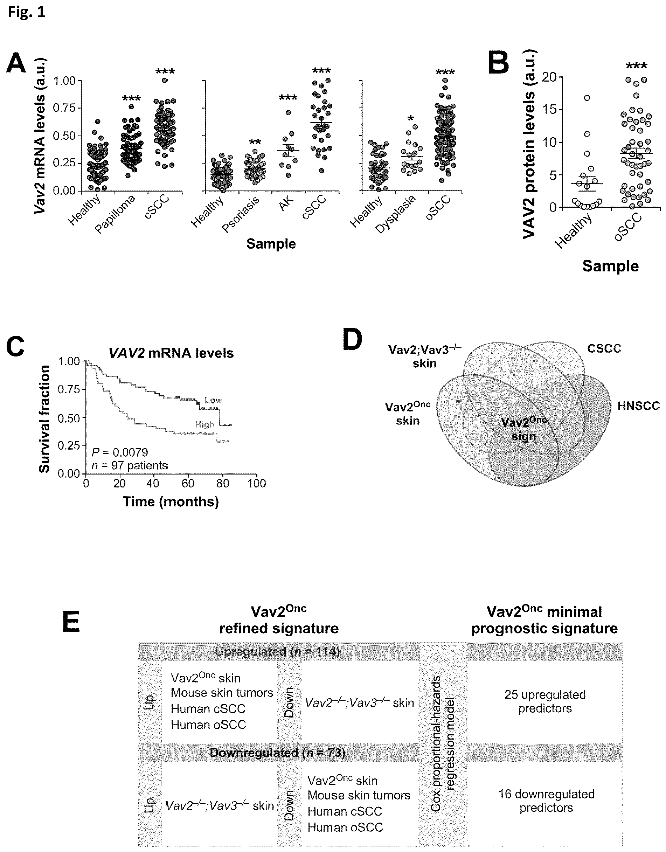

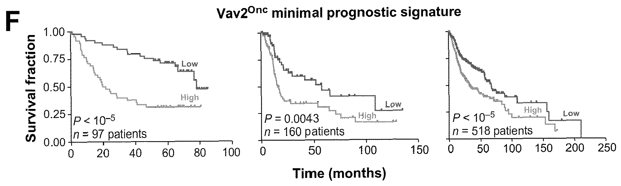

**Figure 1.** In silico analyses of public high-throughput datasets encompassing over 400 samples revealed that *VAV2* is transcriptionally deregulated in SCC.

In mouse models of skin tumorigenesis, this GEF shows a clear overexpression in papilloma and cSCC tumors, especially in the latter (**panel A, left**). Similarly, an upregulation of *VAV2* is detected in patients with actinic keratosis, and such deregulation is magnified in full-blown cSCCs (**panel A, middle**). This finding is not restricted to skin, as head and neck dysplasias and SCCs also display high levels *VAV2* (**panel A, right**). Consistent with this, tumor samples from HNSCC patients display a strong enrichment of VAV2 protein when compared to healthy tissue **(panel** B). The expression level of *VAV2* mRNA can stratify oral SCC patients in low- and high-survival groups with statistical confidence (**panel C**). VAV2 drives the expression of an autonomous gene signature that is consistently conserved in human SCC samples (**panel D**). A Cox proportional-hazards regression model was applied to such VAV2-driven signature to identify a core of 41 genes strongly associated with patient survival (**panel E**). According to the expression levels of such 41-gene signature, patients can be classified into high and low enrichment groups. The belonging to either of these groups robustly predicts patient survival with a very high statistical confidence, as has been validated in over 600 patients (**panel F**).

## DETAILED DESCRIPTION OF THE INVENTION

### Definitions

The terms "subject", or "individual"' are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "subject suspected of having cancer" as used herein, refers to a subject that presents one or more signs or symptoms indicative of a squamous cell carcinoma, including skin (cSCC) and head and neck (HNSCC) carcinomas, and is being screened for said cancer. A subject suspected of having cancer encompasses for instance an individual who has received a preliminary diagnosis *(e.g.*, an X-ray computed tomography scan showing a mass) but for whom a confirmatory test *(e.g.*, biopsy and/or histology) has not been done or for whom the stage of cancer is not known or not known in enough detail.

The term "prognosis" as used herein refers to predicting disease progression or outcome. More specifically, "prognostic markers" may refer to patient or tumor characteristics that predict outcome (usually survival) independent of the treatment. Thus, they are usually identified and validated in patients who receive no therapy or surgical therapy only. The goal of identifying prognostic markers is to define patient subpopulations with significantly different anticipated outcomes, which might benefit from different therapies. Good prognostic patients may not require additional treatment beyond the primary surgical resection, while poor prognostic patients may derive improved survival benefit from adjuvant therapy or other closer clinical follow up or therapeutic strategy.

"Predictive markers", on the other hand, may refer to patient or tumor characteristics that predict benefit from specific treatments (either in terms of tumor shrinkage or survival). In other words, the differences in tumor response or survival benefit between treated versus untreated patients will be significantly different in those positive or negative for the predictive marker (Zhu CQ and Tsao MS, 2014).

The term "therapeutically effective amount" as used herein refers to an amount that is effective, upon single or multiple dose administration to a subject (such as a human patient) in the prophylactic or therapeutic treatment of a disease, disorder or pathological condition.

The term "marker" or "biomarker" as used herein refers to markers of disease, prognostic or predictive markers which are typically substances found in a bodily sample that can be easily measured. Said bodily sample can be for instance a tumor, tissue, blood, plasma or feces sample. The term biomarker encompasses biophysical and biochemical determinations, including genetic and serological markers.

The term "combination therapy" as used throughout the specification, is meant to comprise the administration of the referred therapeutic agents to a subject suffering from cancer, in the same or separate pharmaceutical formulations, and at the same time or at different times. If the therapeutic agents are administered at different times they should be administered sufficiently close in time to provide for the combined effect (e.g. potentiating or synergistic response) to occur. The particular combination of therapies to employ in a combination regimen will take into account compatibility of the desired therapeutics and/or procedures and/or the desired therapeutic effect to be achieved. It will be appreciated that the therapies employed may achieve a desired effect for the same disorder (for example, anticancer effects), and/or they may achieve different effects (e.g., control of any adverse effects).

### Prognostic and predictive methods of the Invention

In a first aspect, the present invention provides an *in vitro* multi-platform method for determining the prognosis of a subject having squamous cell carcinoma, preferably skin (cSCC), head and neck (HNSCC), lung (NSCLC) and thyroid (SCTC) carcinomas, wherein said method comprises:

> a) determining in a biological sample isolated from said subject the gene expression levels of the Rho guanine nucleotide exchange factor *VAV2* and/or, optionally, the gene expression levels of at least one, two, three, four, five and preferably of at least all of the VAV2-induced transcriptional signature markers selected from the group consisting of:

*ABCA9, ACPP, ANXA9, AREG, ARHGAP20, ATP1A2, AURKA, BCAT1, BIRC5, CAMK1D, CCL4, CDCA5, CDCA8, CILP, CKS1B, CXCL2, EXPH5, FSCN1, FYCO1, HAS3, HSPB6, IL24, LAMC2, MMP9, MYC, NEK2, NOLC1, ODC1, PKIB, PYGM, SCEL, SERPINE1, SLC20A1, SLC2A3, SLC6A4, SLC7A11, SLC7A5, SOCS3, SRM, SSBP2, TCEA3;*

b) calculating a combined score from the gene expression levels of the markers determined in the biological sample as defined in step a); and

c) classifying the subject as having good prognosis or poor prognosis based on the combined score.

In a related aspect, it refers to an *in vitro* method for obtaining useful data for the prognosis of a subject having squamous cell carcinoma, said method comprising the steps defined above.

Signs or symptoms which may be indicative of squamous cell carcinoma include for instance one or more of the following: weight loss, loss of appetite, malaise, fever, cough, dyspnea, wheezing, stridor, hoarseness, shortness of breath, weakness, haemoptisis, chest and or back pain, obstructive pneumonia and pleural effusion (see for instance, WHO classification of Tumors of the Lung, Pleura, Thymus and Heart. Edited by WD Travis, E Brambilla, A.P. Burke, A. Marx and A.G. Nicholson (2015).

In the present invention, the expression "a subject having squamous cell carcinoma" may refer to a subject which has been suspected to have or has been diagnosed with squamous cell carcinoma and wherein further to histological analysis (e.g. from a pre-surgery biopsy or a biopsy from the resected tumor) it has been determined that said cancer is squamous cell carcinoma. In a particular embodiment, said subject has been submitted to tumor resection surgery and has not received any neoadjuvant treatment.

The combined score determined by the methods of the present invention may be predictive and/or prognostic.

Disease progression or outcome may be measured using different parameters, including but not limited to, tumor growth, tumor growth delay, increase/decrease of tumor size, increase/decrease in tumor markers, and patient's survival.

Preferably, in the context of the present invention, the clinical outcome of a subject, is expressed as overall survival and/or disease-free survival. Survival of cancer patients is generally suitably expressed by Kaplan-Meier curves, named after Edward L. Kaplan and Paul Meier who first described it (Kaplan, Meier: Amer. Statist. Assn. 53:457-481). The Kaplan-Meier estimator is also known as the product limit estimator. It serves for estimating the survival function from life-time data. A plot of the Kaplan-Meier estimate of the survival function is a series of horizontal steps of declining magnitude which, when a large enough sample is taken, approaches the true survival function for that population. The value of the survival function between successive distinct sampled observations is assumed to be constant. With respect to the present invention, the Kaplan-Meier estimator may be used to measure the fraction of patients living for a certain amount of time after beginning a therapy (e.g. after tumor resection). The clinical outcome predicted may be the (overall/disease-free) survival in months/years from the time point of taking the sample. It may be survival for a certain period from taking the sample, such as of six months or more, one year or more, two years or more, three years or more, four years or more, five years or more, six years or more. In each case, "survival" may refer to "overall survival" or "disease free survival".

The term "disease free survival", or "DFS" as used herein, is defined as the interval of time from start of treatment (e.g., date of surgery) to the first measurement of cancer growth. The term "overall survival" or "OS" as used herein, is defined as the interval of time from the start of treatment (e.g., date of surgery) to death from any cause.

The term "poor prognosis" as used herein refers to a high risk of recurrence and/or death. In preferred embodiments, the term "poor prognosis" means a survival (i.e. DFS and/or OS) of six months or less, one year or less, two years or less, three years or less, four years or less, five years or less, six years or less, etc. In one embodiment, the term poor prognosis refers to a DFS and/or OS of less than 5 years.

**Step (a)** of the method under the first aspect of the invention comprises determining in said biological sample the expression levels of the gene markers defined above.

The term "*VAV2*" as used herein refers to the human gene with the identification number 12658 from the Human Genome Organization Nomenclature Committee (HGNC).

The term "*ABCA9*" as used herein refers to the human gene with the identification number 39 from the Human Genome Organization Nomenclature Committee (HGNC).

The term "*ACPP*" as used herein refers to the human gene with the identification number 125 from the Human Genome Organization Nomenclature Committee (HGNC).

The term "*ANXA9*" as used herein refers to the human gene with the identification number 547 from the Human Genome Organization Nomenclature Committee (HGNC).

The term "*AREG*" as used herein refers to the human gene with the identification number 651 from the Human Genome Organization Nomenclature Committee (HGNC).

The term "*ARHGAP20*" as used herein refers to the human gene with the identification number 18357 from the Human Genome Organization Nomenclature Committee (HGNC).

The term "*ATP1A2*" as used herein refers to the human gene with the identification number 800 from the Human Genome Organization Nomenclature Committee (HGNC).

The term "*AURKA*" as used herein refers to the human gene with the identification number 11393 from the Human Genome Organization Nomenclature Committee (HGNC).

The term "*BCAT1*" as used herein refers to the human gene with the identification number 976 from the Human Genome Organization Nomenclature Committee (HGNC).

The term "*BIRC5*" as used herein refers to the human gene with the identification number 593 from the Human Genome Organization Nomenclature Committee (HGNC).

The term "*CAMK1D*" as used herein refers to the human gene with the identification number 19341 from the Human Genome Organization Nomenclature Committee (HGNC).

The term "*CCL4*" as used herein refers to the human gene with the identification number 10630 from the Human Genome Organization Nomenclature Committee (HGNC).

The term "*CDCA5*" as used herein refers to the human gene with the identification number 14626 from the Human Genome Organization Nomenclature Committee (HGNC).

The term "*CDCA8*" as used herein refers to the human gene with the identification number 14629 from the Human Genome Organization Nomenclature Committee (HGNC).

The term "*CILP*" as used herein refers to the human gene with the identification number 1980 from the Human Genome Organization Nomenclature Committee (HGNC).

The term "*CKS1B*" as used herein refers to the human gene with the identification number 19083 from the Human Genome Organization Nomenclature Committee (HGNC).

The term "*CXCL2*" as used herein refers to the human gene with the identification number 4603 from the Human Genome Organization Nomenclature Committee (HGNC).

The term "*EXPH5*" as used herein refers to the human gene with the identification number 30578 from the Human Genome Organization Nomenclature Committee (HGNC).

The term "*FSCN1*" as used herein refers to the human gene with the identification number 11148 from the Human Genome Organization Nomenclature Committee (HGNC).

The term "*FYCO1*" as used herein refers to the human gene with the identification number 14673 from the Human Genome Organization Nomenclature Committee (HGNC).

The term "*HAS3*" as used herein refers to the human gene with the identification number 4820 from the Human Genome Organization Nomenclature Committee (HGNC).

The term "*HSPB6*" as used herein refers to the human gene with the identification number 26511 from the Human Genome Organization Nomenclature Committee (HGNC).

The term "*IL24*" as used herein refers to the human gene with the identification number 11346 from the Human Genome Organization Nomenclature Committee (HGNC).

The term "*LAMC2*" as used herein refers to the human gene with the identification number 6493 from the Human Genome Organization Nomenclature Committee (HGNC).

The term "*MMP9*" as used herein refers to the human gene with the identification number 7176 from the Human Genome Organization Nomenclature Committee (HGNC).

The term "*MFC*" as used herein refers to the human gene with the identification number 7553 from the Human Genome Organization Nomenclature Committee (HGNC).

The term "*NEK2*" as used herein refers to the human gene with the identification number 7745 from the Human Genome Organization Nomenclature Committee (HGNC).

The term "*NOLC1*" as used herein refers to the human gene with the identification number 15608 from the Human Genome Organization Nomenclature Committee (HGNC).

The term "*ODC1*" as used herein refers to the human gene with the identification number 8109 from the Human Genome Organization Nomenclature Committee (HGNC).

The term "*PKIB*" as used herein refers to the human gene with the identification number 9018 from the Human Genome Organization Nomenclature Committee (HGNC).

The term "*PYGM*" as used herein refers to the human gene with the identification number 9726 from the Human Genome Organization Nomenclature Committee (HGNC).

The term "*SCEL*" as used herein refers to the human gene with the identification number 10573 from the Human Genome Organization Nomenclature Committee (HGNC).

The term "*SERPINE1*" as used herein refers to the human gene with the identification number 8583 from the Human Genome Organization Nomenclature Committee (HGNC).

The term "*SLC20A1*" as used herein refers to the human gene with the identification number 10946 from the Human Genome Organization Nomenclature Committee (HGNC).

The term "*SLC2A3*" as used herein refers to the human gene with the identification number 11007 from the Human Genome Organization Nomenclature Committee (HGNC).

The term "*SLC6A4*" as used herein refers to the human gene with the identification number 11050 from the Human Genome Organization Nomenclature Committee (HGNC).

The term "*SLC7A11*" as used herein refers to the human gene with the identification number 11059 from the Human Genome Organization Nomenclature Committee (HGNC).

The term "*SLC7A5*" as used herein refers to the human gene with the identification number 11063 from the Human Genome Organization Nomenclature Committee (HGNC).

The term "*SOCS3*" as used herein refers to the human gene with the identification number 19391 from the Human Genome Organization Nomenclature Committee (HGNC).

The term "*SRM*" as used herein refers to the human gene with the identification number 11296 from the Human Genome Organization Nomenclature Committee (HGNC).

The term "*SSBP2*" as used herein refers to the human gene with the identification number 15831 from the Human Genome Organization Nomenclature Committee (HGNC).

The term "*TCEA3*" as used herein refers to the human gene with the identification number 11615 from the Human Genome Organization Nomenclature Committee (HGNC).

In a particular embodiment, the prognosis method of the invention comprises determining in step a) the gene expression levels of the Rho guanine nucleotide exchange factor *VAV2*

In a preferred embodiment, the prognosis method of the invention comprises determining in step a) the gene expression levels of at least one, two, three, four, five and preferably of at least all of the VAV2-induced transcriptional signature markers.

By "determining expression" or "gene expression" is meant broadly determining production by transcription of an RNA product or production by translation of a proteinaceous product of the gene. The phases encompass determination of the presence or absence of expression as well as increased or diminished expression relative to a standard or other control as explained below. When comparing gene expression between two sample types, differential expression refers to production of the product at a higher or lower level in one sample compared to the other. Selective expression refers to production of the product in one cell type or sample and not the other. Techniques to assay levels of individual biomarkers from test samples are well known to the skilled technician, and the invention is not limited by the means by which the components are assessed.

Expression levels may be absolute or relative. When the expression levels are normalized, normalization can be performed with respect to different measures in the sample. These procedures are well known to one skilled in the art. Typically, expression levels are normalized with respect to an "endogenous control". An "endogenous control" as used herein relates to a gene expression product whose expression levels do not change or change only in limited amounts in tumor cells with respect to non-tumorigenic cells. "Endogenous control" is usually the expression product from a housekeeping gene and which codes for a protein which is constitutively expressed and carries out essential cellular functions. Housekeeping genes that can be used as endogenous control include for example β-2-microglobulin, ubiquitin, 18-S ribosomal protein, cyclophilin, GAPDH, actin and HPRT.

In some embodiments, the gene-signature expression is expression of RNA.

In some embodiments, the method comprises determining gene signature expression in tumor cells isolated from the sample.

In some embodiments, the gene signature comprises or consists essentially of at least one, two or more genes selected from the group consisting of the genes set out in the VAV2-induced transcriptional signature markers outlined above. In some embodiments the signature comprises between 2 to 10, or 8 to 20 or 15 to 30, more preferably at least the 41 genes.

"Two or more genes selected from the VAV2-induced transcriptional signature " includes 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33,34, 35, 36, 37, 38, 39, 40, or 41 genes from the 41 markers as set out above. Although more than 41 upregulated genes are described in the present specification, this number encompasses the most selectively expressed genes, although the use of more than this number of genes is likely to provide more information. Therefore, use of more than 41 genes is in no way excluded.

In some embodiments of the method, the method comprises detection of RNA expression products using complementary oligonucleotide probes. Thus, the method comprises hybridization to oligonucleotide probes to detect RNA products from a predetermined gene set. Preferably, the gene set comprises one of the herein-described gene sets.

In accordance with the present invention, the level of expression of each gene in the gene signature is used to give a measure of gene expression of the sample for comparison with reference measures. In some embodiments, the measure is a profile or signature of gene expression that may be represented numerically or graphically to facilitate interpretation of the sample data.

The identified signatures preferably consist of an algorithm, which combines the coefficient-weighted expression of the selected marker genes analyzed by any known method useful for the transcriptomic profiling such as expression microarrays. The levels of the expression of each gene are preferably measured by a gene-Score and the algorithm assigns a coefficient to each gene G-Score to get a final combined score that in the context of this invention is called Prognostic Index (PI) value.

In a preferred embodiment of the present invention the Gene score (G-Score) value is determined per biological sample according to the enrichment of the VAV2-induced transcriptional signature as calculated by the single-sample Gene Set Enrichment Analysis (ssGSEA) algorithm. This methodology is described in Barbie DA, Tamayo P, et al. Systematic RNA interference reveals that oncogenic KRAS-driven cancers require TBK1. Nature. 2009;462:108-112.

The final score, called "G-Score", is preferably calculated by computing the combined enrichment score (ES) of the UP fraction ($S_{UP}$, composed of *AREG, AURKA, BCAT1, BIRC5, CCL4, CDCA5, CDCA8, CKS1B, CXCL2, FSCN1, HAS3, IL24, LAMC2, MMP9, MYC, NEK2, NOLC1, ODC1, SERPINE1, SLC20A1, SLC2A3, SLC7A11, SLC7A5, SOCS3* and *SRM*) and the DN fraction ($S_{DN}$, composed of *ABCA9, ACPP, ANXA9, ARHGAP20, ATP1A2, CAMK1D, CILP, EXPH5, FYCO1, HSPB6, PKIB, PYGM, SCEL, SLC6A4, SSBP2* and *TCEA3)* of the VAV2-induced transcriptional signature. The following formula may be applied:

$$G\text{-}Score = ES(G_{UP}, S_{UP}) - ES(G_{DN}, S_{DN})$$

The resulting value of the G-Score determines the combined level of expression of the markers from the VAV2-induced transcriptional signature.

The prognostic method of the invention can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In performing the method of the present invention, said biological sample from the cancer patient is preferably a sample containing tumor cells. Tumors or portions thereof may be surgically resected from the patient or obtained by routine biopsy. Preferably, a tumor sample is obtained from the primary tumor. In a particular embodiment, optionally in combination with one or more of the embodiments or features described above or below, said biological sample isolated from the subject is a tumor biopsy sample, preferably obtained from a resected tumor.

These types of samples are routinely used in the clinical practice and a person skilled in the art will know how to identify the most appropriate means for their obtaining and preservation.

Once a sample has been obtained, it may be used fresh, it may be frozen or preserved using appropriate means (e.g., as a formalin-fixed, paraffin-embedded tissue sample). Such biological samples can be taken around the time of diagnosis, before, during or after treatment (e.g. surgical resection).

**Step (b)** of the prognosis method under the first aspect of the invention comprises calculating a combined score. The combined score is a value obtained according to a given mathematical algorithm wherein the expression values of each of the protein markers used in the methods of the invention are variables of said mathematical algorithm.

In a particular embodiment, when calculating the combined score, this is proportional to the expression levels of one or more of the genes identified in step a), wherein the higher the score, the worst the prognosis and/or the higher the risk of relapse.

Preferably, said combined score is calculated as the combination of the enrichment scores obtained in a single-sample Gene Set Enrichment Analysis for each fraction (UP and DN) of the VAV2-induced transcriptional signature (e.g. expressed as G-Score value). In the context of the invention, the combined score obtained in this way is also named Prognostic Index (PI).

In a preferred embodiment, the combined score is a Prognostic Index obtained by using a formula selected from the group consisting of:

$$PI = ES(G_{UP}, S_{UP}) - ES(G_{DN}, S_{DN})$$

Furthermore, **step (c)** of the method under the first aspect of the invention comprises classifying the subject as having good prognosis or poor prognosis based on the combined score.

Typically, in step c) said method comprises comparing the combined score in the subject sample with a reference combined score; and an increase of the combined score in the subject sample with regard to said reference combined score is indicative of poor prognosis.

The term "reference combined score" as used herein is a reference value obtained according to a given mathematical algorithm wherein reference expression values of each of the gene markers used in the prognosis method of the invention are variables of said mathematical algorithm.

The term "reference value", as used herein, relates to a predetermined criteria used as a reference for evaluating the values or data obtained from the samples collected from a subject. This "reference value" may also be referred as "cut-off value" or "threshold value".

The reference value or reference level can be an absolute value, a relative value, a value that has an upper or a lower limit, a range of values, an average value, a median value, a mean value, a tertile value, or a value as compared to a particular control or baseline value. In a particular embodiment, optionally in combination with one or more of the embodiments or features described above or below, said reference value is the mean value or the tertile value.

A reference value can be based on an individual sample value but is generally based on a large number of samples, including or excluding the sample to be tested. For instance, this reference value may be derived from a collection of tumor tissue samples from a reference SSC population for whom historical information relating to the actual clinical outcome for the corresponding cancer patient is available.

In a particular embodiment, optionally in combination with any of the embodiments described above or below, said combined reference value is determined by a method comprising:

> a) determining, for each SSC patient in a reference population, the gene expression levels of the gene markers as defined in step a) and calculating the combined score as defined in step b) of the method of the invention;
>
> b) selecting as provisional reference value an arbitrary combined score from the ones obtained in step a);
>
> c) classifying the patients in the reference SCC population in two groups according to the provisional reference value selected combined score obtained in a), wherein:
>
> > (i) the first group comprises SCC patients that exhibit a combined score that is lower than the arbitrary/provisional reference value; and
> > (ii) the second group comprises SCC patients that exhibit a combined score that is higher than the arbitrary/provisional reference value;
>
> whereby two groups of SCC patients are obtained for the specific reference value
>
> d) statistically correlating the combined score with the clinical follow up data (disease free survival and overall survival) using log rank test.

In the prognosis method of the invention, the combined score obtained in step b) is considered "decreased" when said combined score is lower than a reference combined score. Preferably, the combined score is considered to be lower than a reference combined score when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than the reference combined score.

Likewise, in the context of the prognosis method of the invention, the combined score obtained in step b) is considered "increased" when said combined score is higher than a reference value. Preferably, the combined score is considered to be higher than a reference combined score when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference combined score.

Alternatively or in addition, subjects having more than about 1.1,1.2, 1.3, 1.4, 1.5, 2, 3, 4, 5, 6, 7, 8, 9, 10, 15 or 20 fold levels deviation (i.e., increase or decrease) than the reference combined score as described herein.

Prognosis or outcome prediction in the method of the invention, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical tools; illustrative, non-limiting examples of said statistical tools include determining confidence intervals, determining the p-value, the Chi-Square test discriminating functions, etc. Preferred confidence intervals are at least 90%, at least 95%, at least 97%, at least 98%, at least 99%. The p-values are, preferably less than 0.1, less than 0.05, less than 0.01, less than 0.005 or less than 0.0001. The teachings of the present invention preferably allow correctly classifying at least 60%, at least 70%, at least 80%, or at least 90% of the subjects of a determining group or population analyzed.

It is further noted that the accuracy of the method of the invention can be further increased by determining the presence and/or quantification of other prognostic/predictive molecular markers (Coate LE et al, 2009; Ferte C et al 2010; Lin J and Beer DG, 2012; Zhu CQ and Tsao MS, 2014), and/or clinical signs or symptoms with reported prognostic/predictive value, such as morphological features of the tumor, histological subtypes, radiological traits of the imaging tests (e.g. size, shape, volume, radiological texture, morphological details or other features in a CT scan, X-Ray or SUV or alternative ways to analyze nuclear tracer levels in a PET imaging, etc); clinical characteristics of the patients (e.g. age, sex, race, respiratory function levels, performance status). The potential additional molecular markers to be associated to the present invention can be found in the tumor specimen itself or other cells, body fluids or exhaled breath obtained from the same patient.

The methods of the present invention or any of the steps thereof might be implemented by a computer. Therefore, a further aspect of the invention refers to a computer implemented method, wherein the method is any of the methods disclosed herein or any combination thereof.

It is noted that any computer program capable of implementing any of the methods of the present invention or used to implement any of these methods or any combination thereof, also forms part of the present invention.

This computer program is typically directly loadable into the internal memory of a digital computer, comprising software code portions for performing the steps of comparing the combined score (e.g., obtained from the level of one or more of the target markers as described in the invention), from the one or more biological samples of a subject with a reference value (e.g., reference combined value) and determining the prognosis of said subject or whether it would benefit from adjuvant therapy, when said product is run on a computer.

It is also noted that any device or apparatus comprising means for carrying out the steps of any of the methods of the present invention or any combination thereof, or carrying a computer program capable of, or for implementing any of the methods of the present invention or any combination thereof, is included as forming part of the present specification.

The methods of the invention may also comprise the storing of the method results in a data carrier, preferably wherein said data carrier is a computer readable medium. The present invention further relates to a computer-readable storage medium having stored thereon a computer program of the invention or the results of any of the methods of the invention.

As used herein, "a computer readable medium" can be any apparatus that may include, store, communicate, propagate, or transport the results of the determination of the method of the invention. The medium can be an electronic, magnetic, optical, electromagnetic, infrared, or semiconductor system (or apparatus or device) or a propagation medium.

**Other methods of the invention**

In another aspect, the present invention provides an *in vitro* method for selecting those subjects having SCC who are expected to benefit from an adjuvant treatment, wherein said method comprises:

> a) classifying said subject according to the prognosis method as described herein;
> b) deciding on whether to administer an adjuvant treatment according to the prognosis classification;

wherein a subject classified as having poor prognosis will be selected for administration of an adjuvant treatment.

The term "adjuvant treatment" as used herein refers to a treatment administered after surgical resection of a tumor, typically the primary tumor. Said adjuvant treatment may comprise the administration of any cytotoxic or antiproliferative drug and includes chemotherapy and/or targeted therapies regimens. For instance, said adjuvant treatment may be selected from (but it is not limited to) any one or more of the group consisting of platinum anticancer agents (e.g., cisplatin, oxaliplatin, carboplatin, BBR3464, satraplatin, tetraplatin, ormiplatin, and iproplatin); antimetabolites (e.g., 5-fluorouracil, gemcitabine, cytarabine, capecitabine, decitabine, floxuridine, 6-mercaptopurine, methotrexate, fludarabine, aminopterin, pemetrexed, raltitrexed, cladribine, clofarabine, fludarabine, mercaptopurine, pentostatin, and thioguanine); mitotic inhibitors (e.g., paclitaxel, docetaxel, vinblastine, vincristine, vindesine, and vinorelbine); anthracycline antibiotics (e.g., bleomycin, daunorubicin, doxorubicin, epirubicin, idarubicin, mitomycin, mitoxantrone, pixantrone, and valrubicin); topoisomerase I and/or II inhibitors (e.g., topotecan, SN-38, irinotecan, camptothecine, rubitecan, etoposide, and teniposide); antitumor monoclonal antibodies (e.g., bevacizumab, cetuximan, panitumumab, trastuzumab, rituximab, tositumomab, alemtuzumab, and gemtuzumab); tyrosine kinase inhibitors (e.g., erlotinib, sorafenib, axitinib, bosutinib, cediranib, dasatinib, gefitinib, imatinib, canertinib, lapatinib, lestaurtinib, nilotinib, semaxanib, sunitinib, and vandetanib);.metabolic modulators (e.g., mTOR inhibitors), epigenetic inhibitors (e.g., DNMT inhibitors) and immunotherapy agents (e.g. Pembrolizumab, Nivolumab, Atezolizumab, Avelumab).

Preferably said adjuvant therapy comprises the administration of a platinum anticancer agent as single agent or in a combination therapy. Preferably, the platinum anticancer agent is cisplatin and/or carboplatin.

In a further aspect, the present invention refers to an *in vitro* method for predicting the efficacy of an adjuvant treatment (e.g., adjuvant chemotherapy) in a subject having SCC, wherein said method comprises:

> a) classifying said subject according to the prognosis method as described herein; and
> b) predicting the efficacy of said adjuvant treatment (e.g. adjuvant chemotherapy) according to the prognosis classification;

preferably wherein classification of the subject as having poor prognosis is indicative of increased efficacy of the adjuvant treatment (e.g. adjuvant chemotherapy).

Preferably, said adjuvant treatment comprises the administration of a platinum anticancer agent, and a poor prognosis is indicative of increased efficacy of the platinum anticancer agent.

In another further aspect, the present invention refers to an i*n vitro* method for selecting an adjuvant treatment for a subject having SCC, wherein said method comprises:

> a) classifying said subject according to the prognosis method as described herein; and
> b) selecting an adjuvant treatment (preferably an adjuvant chemotherapy treatment) according to the prognosis classification.

Preferably, when the subject is classified as having poor prognosis is an adjuvant chemotherapy (preferably, a platinum anticancer agent) is selected.

In an additional aspect, the present invention provides a method for treating a subject having SCC comprising administering to said patient a therapeutically effective amount of a treatment, typically an adjuvant treatment, wherein said treatment is selected according to the classification of said patient according to the prognosis method of the invention. Preferably, when said patient is classified as having poor prognosis the administered treatment comprises a platinum anticancer agent.

**Kit and use of a kit in the methods of the invention**

A further aspect, the present invention refers to a kit for determining the levels of one or more of the target markers as described herein in a biological sample (preferably a tumor biopsy sample) isolated from a subject. The kit may also contain instructions indicating how the materials within the kit may be used.

The term "kit" or "testing kit" denotes combinations of reagents and adjuvants required for an analysis. Although a test kit consists in most cases of several units, one-piece analysis elements are also available, which must likewise be regarded as testing kits.

The present invention thus further provides a prognostic probe set or kit comprising same comprising one, two or more polynucleotides each capable of selectively hybridizing to mRNA of one of the genes in the signature of genes defined hereinbefore.

In some embodiments, the probe set comprising one or two or more polynucleotides each capable of hybridizing to mRNA of KIT gene.

It is contemplated that any features described herein can optionally be combined with any of the embodiments of any method, kit, use of a kit, or computer program of the invention; and any embodiment discussed in this specification can be implemented with respect to any of these. It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of' and "consists essentially of'. As used herein, the phrase "consisting essentially of' limits the scope of a claim to the specified materials or steps and those that do not materially affect the basic and novel characteristic(s) of the claimed invention. As used herein, the phrase "consisting of' excludes any element, step, or ingredient not specified in the claim except for, e.g., impurities ordinarily associated with the element or limitation.

The term "or combinations thereof' as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB.

Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

The following examples serve to illustrate the present invention and should not be construed as limiting the scope thereof.

**EXAMPLES**

**MATERIALS AND METHODS**

R version 3.5.1 was used for the bioinformatic analyses along with Perl for text processing. Signal intensity values were obtained from CEL files after robust multichip average (RMA). Differentially expressed genes were identified using linear models for microarray data (Limma). Adjusted P-values for multiple comparisons were calculated applying the Benjamini-Hochberg correction (FDR). GEO datasets GSE45216 and GSE13355 (human cutaneous SCC), GSE30784 (human oral SCC) and GSE21264 (mouse cutaneous SCC) where used to obtain *Vav2* mRNA expression levels according to pathological status. Survival analyses were performed through Kaplan-Meier estimates of overall survival according to the expression level of *VAV2* using the GSE41613 dataset. The median of the *VAV2* expression distribution was used to establish the low and high expression groups and, subsequently, the Mantel-Cox test was applied to statistically validate the differences between the survival distributions. To evaluate the similarity between the transcriptional programs activated by Vav2 and the ones driven by SCC tumors, the datasets GSE45216 and GSE13355 (human cutaneous), GSE30784 (human oral) and GSE21264 (mouse cutaneous) were used. To assess conservation of the Vav2-associated transcriptional signature across tumors, a 1.5 fold change threshold was used. To evaluate the enrichment of the VAV2-associated gene signature across SCC tumors, single-sample Gene Set Enrichment Analysis was used. For the identification of the VAV2-driven prognostic signature, the Cox proportional-hazards regression model was applied using the survival R package. Survival analyses according to the VAV2-driven prognostic signature enrichment were performed as indicated above, using datasets from the GEO (GSE41613) and the TCGA (HNSCC collection).

**RESULTS VAV2**

- **VAV2 is upregulated in SCC and associated with survival**

In silico analyses of public high-throughput datasets encompassing over 400 samples revealed that *VAV2* was transcriptionally deregulated in SCC. In mouse models of skin tumorigenesis, this GEF showed a clear overexpression in papilloma and cSCC tumors, especially in the latter (**panel A, left**). Similarly, an upregulation of *VAV2* was detected in patients with actinic keratosis, and such deregulation was magnified in full-blown cSCCs (**panel A, middle**). This finding was not restricted to skin, as head and neck dysplasias and SCCs also displayed high levels *VAV2* (**panel A, right**). In order to validate this in silico data, we extracted total protein from 63 HNSCC patient samples, immunoprecipitated VAV2, and assessed its abundance through Western blot. This approach allowed us to confirm that tumor samples displayed a strong enrichment of VAV2 protein when compared to healthy tissue (**panel B**).

Interestingly, we found a positive correlation between VAV2 expression and clinical stage (data not shown). This association led us to wonder whether VAV2 levels were associated with HNSCC patient survival. Based on public clinical data, our analyses revealed that the expression level of this GEF could stratify patients in low- and high-survival groups with statistical confidence (**panel C**). We deemed that this could reflect the activation of a VAV2-driven molecular program with a prominent role in squamous cell carcinoma. To test this hypothesis we used a Vav2$^{Onc}$ knock-in mouse model, which expressed a constitutively active version of *Vav2*.

- **Vav2 controls a SCC-associated transcriptional signature**

Examination of Vav2$^{Onc}$ mice revealed that the constitutive activation of this GEF leads to defects in squamous epithelia, such as the skin. Through transcriptomic profiling based on expression microarrays, we found that Vav2 indeed drives a vast transcriptional program. Interestingly, this Vav2-induced transcriptional signature is primarily composed of genes involved in proliferation and in the maintenance of a stem-like state. In order to refine this signature, we decided to identify those genes that were more strongly associated to Vav2 function in a squamous cell carcinoma context. Firstly, to establish the genes that were more strongly dependent on Vav2, we compared the Vav2$^{Onc}$ and the Vav2$^{-/-}$Vav3$^{-/-}$ skin transcriptomes, and selected the transcripts that showed a reverse behavior between the two models. Next, using public high-throughput data from over 300 cSCC and HNSCC patients, we established the fraction of the Vav2-driven signature that was consistently present in SCC when compared to healthy tissue (**panel D**). This bioinformatic approach led us to the establishment of the Vav2$^{Onc}$ 'refined' signature, integrated by a group of 187 transcripts.

**RESULTS minimal Vav2-induced transcriptional signature**

The consistent up- or downregulation of a gene in a tumor does not necessarily entail an impact on overall patient survival. Therefore, in order to extract a gene signature with prognostic value, we applied a Cox proportional-hazards regression model (Zhang, 2002) to the genes driven by VAV2 that are conserved across tumors. This allowed us to identify those genes that were more strongly associated with the outcome of the disease and the survival time. As a result, we were able to establish a Vav2$^{Onc}$ minimal prognostic signature, composed of just 41 genes (**panel E**). In order to validate the prognostic value of our 41-gene signature, we used different datasets containing clinical information from the Gene Expression Omnibus (GEO) and The Cancer Genome Atlas (TCGA) databases. For each patient, the signature fit score was calculated by performing independent single-sample Gene Set Enrichment Analysis (ssGSEA) of the up- and downregulated components of the signature (Subramanian et al., 2005). After correcting for gene set size, the difference between both ssGSEA-derived enrichment scores yielded the prognostic signature fit score. In every dataset used we found that the score of our signature consistently predicted survival with strong statistical confidence (**panel F**). All together, its prognostic value was validated in over 600 HNSCC patients, thus proving its utility to reliably stratify patients into risk groups.

**Claims**

1. A in vitro method for determining the prognosis of a subject having squamous cell carcinoma, wherein said method comprises:

    a) determining in a biological sample isolated from said subject the gene expression levels of the Rho guanine nucleotide exchange factor *VAV2* and/or, optionally, the gene expression levels of at least one, two, three, four, five and preferably of at least all of the VAV2-induced transcriptional signature markers selected from the group consisting of: *ABCA9, ACPP, ANXA9, AREG, ARHGAP20, ATP1A2, AURKA, BCAT1, BIRC5, CAMK1D, CCL4, CDCA5, CDCA8, CILP, CKS1B, CXCL2, EXPH5, FSCN1, FYCO1, HAS3, HSPB6, IL24, LAMC2, MMP9, MYC, NEK2, NOLC1, ODC1, PKIB, PYGM, SCEL, SERPINE1, SLC20A1, SLC2A3, SLC6A4, SLC7A11, SLC7A5, SOCS3, SRM, SSBP2* and *TCEA3.*
    b) calculating a combined score from the gene expression levels of the markers determined in the biological sample as defined in step a); and
    c) classifying the subject as having good prognosis or poor prognosis based on the combined score.

2. The prognosis method according to claim 1, wherein in step c) said method comprises comparing the combined score in the subject sample with a reference combined score; and wherein an increase of the combined score in the subject sample with regard to said reference combined score is indicative of poor prognosis.

3. The prognosis method according to any of claims 1 or 2, wherein the term prognosis refers to disease free survival (DFS) and/or overall survival (OS).

4. The prognosis method according to any of claims 1 to 3, wherein said method comprises determining in step a) the gene expression levels of: *ABCA9, ACPP, ANXA9, AREG, ARHGAP20, ATP1A2, AURKA, BCAT1, BIRC5, CAMK1D, CCL4, CDCA5, CDCA8, CILP, CKS1B, CXCL2, EXPH5, FSCN1, FYCO1, HAS3, HSPB6, IL24, LAMC2, MMP9, MYC, NEK2, NOLC1, ODC1, PKIB, PYGM, SCEL, SERPINE1, SLC20A1, SLC2A3, SLC6A4, SLC7A11, SLC7A5, SOCS3, SRM, SSBP2* and *TCEA3.*

5. The prognosis method according to any of claims 1 to 4, wherein said biological sample isolated from the subject is a tumor biopsy sample, preferably obtained from a resected tumor.

6. The prognosis method according to any of claims 1 to 5, wherein in step a) gene expression levels are determined by an expression microarray.

7. The prognosis method according to any of claims 1 to 6, wherein said method is a computer-implemented method.

8. A data-processing apparatus comprising means for carrying out the steps of a method of claim 7.

9. A computer program comprising instructions which, when the program is executed by a computer, cause the computer to carry out the steps of the method of claim 7.

10. A computer-readable storage medium having stored thereon a computer program according to claim 9.

11. *In vitro* method for selecting those subjects having SCC who are expected to benefit from an adjuvant treatment, wherein said method comprises:

    a) classifying said subject according to the prognosis method as defined in any of claims 1 to 6;
    b) selecting for administration of an adjuvant treatment a subject classified in step a) as having poor prognosis.

12. The method according to claim 11, wherein said adjuvant treatment comprises the administration of a platinum anticancer agent.

13. *In vitro* method for predicting the efficacy of an adjuvant chemotherapy in a subject having SCC, wherein said method comprises:

    a) classifying said subject according to the prognosis method as defined in any of claims 1 to 6; and
    b) predicting the efficacy of said adjuvant chemotherapy according to the prognosis classification;

wherein classification of the subject in step a) as having poor prognosis is indicative of increased efficacy of the adjuvant chemotherapy.

14. The method according to claim 13, wherein said adjuvant chemotherapy comprises the administration of a platinum anticancer agent, and wherein a poor prognosis is indicative of increased efficacy of the platinum anticancer agent.

15. *In vitro* method for selecting an adjuvant treatment for a subject having SCC, wherein said method comprises:

> a) classifying said subject according to the prognosis method as defined in any of claims 1 to 6; and
> b) selecting an adjuvant treatment according to the prognosis classification;

wherein when the subject is classified in step a) as having poor prognosis then adjuvant chemotherapy is selected as adjuvant treatment.

16. The method according to claim 15, wherein when the subject is classified as having poor prognosis then an adjuvant treatment comprising the administration of a platinum anticancer agent is selected.

17. Use of a prognostic probe set or kit comprising same comprising one, two or more polynucleotides each capable of selectively hybridizing to mRNA of one of the genes in the signature of genes defined in claim 1, for implementing at least step a) of any of the methods of any of claims 1 to 16.

**Fig. 1**

**F** Vav2$^{Onc}$ minimal prognostic signature

Survival fraction — Time (months)

$P < 10^{-5}$
$n = 97$ patients

$P = 0.0043$
$n = 160$ patients

$P < 10^{-5}$
$n = 518$ patients

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LAI STEPHEN Y ET AL: "Activated Vav2 modulates cellular invasion through Rac1 and Cdc42 in oral squamous cell carcinoma", ORAL ONCOLOGY, vol. 44, no. 7, July 2008 (2008-07), pages 683-688, XP022763328, ISSN: 1368-8375 * the whole document * | 1-15 | INV. C12Q1/6886 |
| Y | V. PATEL ET AL: "Persistent activation of Rac1 in squamous carcinomas of the head and neck: evidence for an EGFR/Vav2 signaling axis involved in cell invasion", CARCINOGENESIS., vol. 28, no. 6, 1 January 2007 (2007-01-01), pages 1145-1152, XP055594599, GB ISSN: 0143-3334, DOI: 10.1093/carcin/bgm008 * the whole document * | 1-15 | |
| Y | CN 108 424 970 A (SHENZHEN YIKANG BIOLOGICAL TECH CO LTD) 21 August 2018 (2018-08-21) * the whole document * | 1-15 | TECHNICAL FIELDS SEARCHED (IPC) C12Q |
| Y | JP 2006 094733 A (ONE CORP AS) 13 April 2006 (2006-04-13) * the whole document * | 1-15 | |

-/--

The present search report has been drawn up for all claims

| Place of search | Date of completion of the search | Examiner |
|---|---|---|
| The Hague | 12 June 2019 | Botz, Jürgen |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 38 2912

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RUGGIERO C ET AL: "VAV2: A novel prognostic marker and a druggable target for adrenocortical carcinoma", ONCOTARGET 2017 IMPACT JOURNALS LLC USA, vol. 8, no. 51, 2017, pages 88257-88258, XP002791956, ISSN: 1949-2553 * the whole document * | 1-15 | |
| A | WO 2012/013931 A1 (QUEEN MARY & WESTFIELD COLLEGE [GB]; TEH MUY TECK [GB]) 2 February 2012 (2012-02-02) * the whole document * | 1-15 | |
| A | GUTTMAN D ET AL: "Expression of MMP-9, TIMP-1, CD-34 and factor-8 as prognostic markers for squamous cell carcinoma of the tongue", ORAL ONCO, ELSEVIER SCIENCE, OXFORD, GB, vol. 40, no. 8, 1 September 2004 (2004-09-01), pages 798-803, XP004525205, ISSN: 1368-8375, DOI: 10.1016/J.ORALONCOLOGY.2004.01.006 * the whole document * | 1-15 | |
| A | LI YADONG ET AL: "AURKA is a predictor of chemotherapy response and prognosis for patients with advanced oral squamous cell carcinoma", TUMOR BIOLOGY, KARGER, BASEL, CH, vol. 36, no. 5, 30 December 2014 (2014-12-30), pages 3557-3564, XP036218314, ISSN: 1010-4283, DOI: 10.1007/S13277-014-2992-8 [retrieved on 2014-12-30] * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 12 June 2019 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 38 2912

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/093379 A1 (HUTCHINSON FRED CANCER RES [US]; UNIV WASHINGTON [US] ET AL.) 19 August 2010 (2010-08-19) * the whole document * | 1-15 | |

TECHNICAL FIELDS
SEARCHED      (IPC)

The present search report has been drawn up for all claims

| Place of search | Date of completion of the search | Examiner |
|---|---|---|
| The Hague | 12 June 2019 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 666 906 A1**

## ANNEX TO THE EUROPEAN SEARCH REPORT
## ON EUROPEAN PATENT APPLICATION NO.

EP 18 38 2912

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2019

| Patent document cited in search report | | Publication date | Patent family member(s) | Publication date |
|---|---|---|---|---|
| CN 108424970 | A | 21-08-2018 | NONE | |
| JP 2006094733 | A | 13-04-2006 | NONE | |
| WO 2012013931 | A1 | 02-02-2012 | NONE | |
| WO 2010093379 | A1 | 19-08-2010 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- WHO classification of Tumors of the Lung. **PLEURA.** Thymus and Heart. 2015
- **KAPLAN ; MEIER.** *Amer. Statist. Assn.,* vol. 53, 457-481
- **BARBIE DA ; TAMAYO P et al.** Systematic RNA interference reveals that oncogenic KRAS-driven cancers require TBK1. *Nature,* 2009, vol. 462, 108-112